Europäisches Patentamt

European Patent Office

Office européen des brevets

(11) Veröffentlichungsnummer: **0 306 859**
**A1**

## (12) EUROPÄISCHE PATENTANMELDUNG

(21) Anmeldenummer: 88114386.1

(22) Anmeldetag: 02.09.88

(51) Int. Cl.4: **G01N 33/84 , G01N 31/22**

(30) Priorität: 03.09.87 DE 3729502

(43) Veröffentlichungstag der Anmeldung:
15.03.89 Patentblatt 89/11

(84) Benannte Vertragsstaaten:
AT BE CH DE ES FR GB GR IT LI LU NL SE

(71) Anmelder: BOEHRINGER MANNHEIM GMBH
Patentabteilung, Abt. E Sandhofer Strasse
112-132 Postfach 31 01 20
D-6800 Mannheim 31 Waldhof(DE)

(72) Erfinder: Siedel, Joachim, Dr.
Weilheimer Strasse 10
D-8139 Bernried(DE)
Erfinder: Schellong, Lieselotte
Hallberger Allee 8
D-8132 Tutzing(DE)
Erfinder: Staepels, Johnny
An der Breite 7
D-8031 Seefeld(DE)
Erfinder: Herrmann, Uwe, Dr.
Wettersteinstrasse 4
D-8139 Bernried(DE)
Erfinder: Town, Michael-Harold, Dr.
Waldstrasse 45
D-8125 Oberhausen(DE)

(74) Vertreter: Huber, Bernhard, Dipl.-Chem. et al
Möhlstrasse 22 Postfach 860 820
D-8000 München 86(DE)

(54) Verfahren und Reagenz zur Bestimmung von Eisen.

(57) Zur Bestimmung von Eisen in Serum durch Freisetzung des gebundenen Eisens, Reduktion zu $Fe^{2+}$, Zugabe eines für den Nachweis von Eisen geeigneten Farbsystems und photometrische Messung in einer tensidhaltigen Probelösung, setzt man der Probenlösung Fettsäure-Polyethylenglykol-Ester, Alkanol-Polyglykol-Ether und mindestens 1 Mol/l Guanidiniumhydrochlorid zu.

FIG.1

EP 0 306 859 A1

## Verfahren und Reagenz zur Bestimmung von Eisen

Die Erfindung betrifft ein Verfahren zur Bestimmung von Eisen in Körperflüssigkeiten durch Freisetzung des gebundenen Eisens, Reduktion zu $Fe^{2+}$, Zugabe eines für den Nachweis von Eisen geeigneten Farbsystems und photometrische Messung in einer tensidhaltigen Probelösung, sowie ein dafür geeignetes Reagenz.

Eisen-Stoffwechselstörungen, insbesondere Eisenmangel und Eisenresorptionsstörungen sind vor allem in der weiblichen Bevölkerung weit verbreitet. Der Nachweis von Eisen in Körperflüssigkeiten, insbesondere im Serum gehört daher zu den Standardbestimmungen in der medizinischen Analytik. Eisen wird mit der Nahrung zugeführt und über die Schleimhaut des Darmes aufgenommen. An Transferrin in dreiwertigem Zustand gebunden wird es dann zum Knochenmark transportiert, wo es hauptsächlich in das Hämoglobin eingebaut wird. Wird zu wenig Eisen aufgenommen, so kommt es zu anämischen Erscheinungen.

Die Bestimmung des Eisens im Serum gehört zu den am häufigsten durchgeführten Spurenelementanalysen in der klinischen Diagnostik. Hierzu sind verschiedene Verfahren bekannt. So ist in Clin. Chem. 26, (1980) 327-331 ein Verfahren beschrieben, bei dem das an Transferrin in Form eines Carbonatkomplexes gebundene, dreiwertige Eisen in stark saurem Milieu freigesetzt wird. Nachteil dieses Verfahrens ist es, daß die stark sauren Reagenzien ätzende und korrodierende Eigenschaften aufweisen. Um diesen Nachteil auszuräumen, ist es beispielsweise aus Clin. Chem. 23, (1977) 237-240 und aus Z. klin. Chem. 3 (1965) 96-99 bekannt, Protein denaturierende Agenzien wie konzentriertes Guanidinhydrochlorid oder anionische Detergenzien zur Freisetzung des Eisens zu verwenden. Diese Verfahren eignen sich jedoch nicht, die Bestimmung von Eisen in triglyzeridreichen, als lipämisch bezeichneten Seren durchzuführen, da weder Guanidinhydrochlorid noch die anionischen Detergenzien eine ausreichende Aufklarungskraft für diese Seren besitzen. Da das Eisen nur in sehr geringer Konzentration im Serum vorkommt, muß aus meßtechnischen Gründen das Verhältnis von Probe zu Reagenzvolumen sehr hoch sein, üblicherweise 1:5 und höher, so daß die zum Teil starken Trübungen im Testansatz eine photometrische Messung verhindern oder aber die Messung verfälschen können.

Zur Lösung dieses Problems wurde in EP 130 537 vorgeschlagen, eine Mischung aus nichtionischen und anionischen Detergenzien zur Freisetzung des Eisens im schwach sauren Medium zu verwenden. Es wurde jedoch gefunden, daß- bei Verwendung dieser Detergenzien zwar rasch und vollständig eine Aufklarung von lipämischen Seren erfolgt, aber für Seren mit einem erhöhten Gehalt an Immunglobulin (Gammopathie-Seren) eine Eintrübung beobachtet wird, die das Meßergebnis verfälschen kann. Zudem ist die Wiederfindung bei stark hämolytischen Seren nicht völlig befriedigend.

Es bestand daher die Aufgabe, ein Verfahren und ein Reagenz zur Bestimmung von Eisen in Serum zur Verfügung zu stellen, bei dem einerseits nicht aggressive Bestandteile verwendet werden müssen und andererseits eine rasche, vollständige und dauerhafte Aufklarung auch stark lipämischer Seren erreicht wird, ohne daß die Messung durch hämolytische Proben oder Seren mit erhöhtem Immunglobulingehalt gestört wird.

Aus Gründen der Automatisierbarkeit und Rationalisierung ist es wünschenswert, dieses Verfahren durchzuführen, ohne daß eine vorhergehende Enteiweißung der Probe erforderlich ist.

Diese Aufgabe wird gelöst durch ein Verfahren zur Bestimmung von Eisen in Serum durch Freisetzung des gebundenen Eisens, Reduktion zu $Fe^{2+}$, Zugabe eines für den Nachweis von Eisen geeigneten Farbsystems und photometrische Messung in einer tensidhaltigen Probelösung, welches dadurch gekennzeichnet ist, daß man der Probenlösung Fettsäure-Polyethylenglykol-Ester, Alkanol-Polyglykol-Ether und mindestens 1 Mol/l Guanidinhydrochlorid zusetzt.

Überraschenderweise wurde festgestellt, daß ein Zusatz der erfindungsgemäßen Kombination aus Fettsäurepolyethylenglykolester, Alkanolpolyglykolether und Guanidiniumhydrochlorid zu einer dauerhaften und vollständigen Aufklarung auch lipämischer Seren führt, ohne daß bei Gammopathie-Seren Wiedereintrübungen auftreten und bei hämolytischen Proben Störungen beobachtet werden.

Zur Bestimmung von Eisen im Serum wird der Probelösung Fettsäurepolyethylenglykolester, Alkanolpolyglykolether und mindestens 1 Mol/l Guanidinhydrochlorid zugesetzt. Dadurch wird das Eisen von seinem Transportprotein, dem Transferrin, freigesetzt.

Als Fettsäurepolyethylenglykolester werden bevorzugt Ester eines Polyethylenglykol mit einem durchschnittlichen Molekulargewicht im Bereich von 200 bis 600 (im folgenden auch als PEG 200 bzw. PEG 600 bezeichnet), bei denen eine OH-Gruppe mit einer linearen Fettsäure verestert ist, während die andere OH-Gruppe entweder frei ist, oder aber mit einer kurzkettigen Säure, wie beispielsweise Essigsäure, verestert ist. Als lineare Fettsäuren sind insbesondere die gesättigten Fettsäuren mit 10 bis 14 C-Atomen im Molekül geeignet. Besonders bevorzugt wird Polyethylenglykolmonolaurat eingesetzt.

Der Fettsäurepolyethylenglykolester wird vorzugsweise in einer Konzentration von 2,5 bis 15 %, besonders bevorzugt 5 bis 10 %, bezogen auf die Probelösung, verwendet.

Als zweite Komponente wird ein Alkanolpolyglykolether eingesetzt. Hier kommen Polyglykole in betracht, deren OH-Gruppen mit linearen oder verzweigtkettigen Alkanolen verethert sind. Bevorzugt werden Polyglykole verwendet, die durchschnittlich 3 bis 6 Glykoleinheiten haben. Die Alkanole haben bevorzugt 8 bis 12 C-Atome in der Kette. Besonders bevorzugt werden Polyglykolether von verzweigtkettigen Alkanolen verwendet. Insbesondere bevorzugt wird ein Ether aus einem Polyglykol mit 4 Glykoleinheiten und einem Isodecanol verwendet.

Der Alkanolpolyglykolether wird bevorzugt in einer Konzentration von 0,5 bis 5 %, insbesondere 1 bis 3 %, eingesetzt.

Weiterhin wird der Probelösung noch Guanidinhydrochlorid zugesetzt. Die Konzentration an Guanidinhydrochlorid sollte relativ hoch sein, wobei mindestens ein Mol/l Guanidinhydrochlorid in der Probelösung vorliegen muß. Bei niedrigeren Konzentrationen an Guanidinhydrochlorid wird keine ausreichende und genügend schnelle Ablösung des Eisens vom Transferrin erreicht. Bis zu 6 Mol/l Guanidinhydrochlorid können bei dem erfindungsgemäßen Verfahren verwendet werden. Eine größere Menge an Guanidinhydrochlorid führt zu keiner weiteren Verbesserung mehr und ist daher unwirtschaftlich. Bevorzugt wird das Guanidiniumhydrochlorid in einem Bereich von 3 bis 5 Mol/l eingesetzt.

Zur Durchführung des erfindungsgemäßen Verfahrens wird die Probelösung bevorzugt in einem schwach sauren Bereich, besonders bevorzugt im Bereich zwischen pH 5 und 6 abgepuffert. Als Puffersubstanzen sind Verbindungen geeignet, die einen pK-Wert von 5 bis 6 aufweisen. Es können jedoch nur Verbindungen verwendet werden, die Eisen nicht komplexieren. Geeignet sind beispielsweise Acetat-, Phosphat-, Succinat- und Tris-Puffer. Bevorzugt wird als Puffersubstanz Acetatpuffer eingesetzt. Der Puffer wird bevorzugt in einer Konzentration von 20 bis 500 mmol/l, besonders bevorzugt 50 bis 150 mmol/l verwendet.

Zur Bestimmung des Eisens wird dann in an sich bekannter Weise der Probelösung ein Reduktionsmittel wie z. B. Ascorbinsäure zugesetzt, um das in dreiwertiger Form vorliegende freigesetzte Eisen in die zweiwertige Form zu reduzieren. Weiterhin wird ein für den Nachweis von Eisen geeignetes Farbsystem zugegeben. Hier sind insbesondere Komplexbildner vom Ferrointyp geeignet, die mit Eisen einen Farbstoff liefern, der photometrisch ausgewertet werden kann. Als geeignete Substanzen sind Bathophenanthrolin und 3-(2-pyridyl)-5,6-bis(4-phenylsulfonsäure)-1,2,4-triazin-dinatriumsalz zu nennen. Die Farbstoffbildung erfolgt dabei proportional zum Eisengehalt der Probe und kann in an sich bekannter Weise photometrisch ausgewertet werden.

Durch die Verwendung der erfindungsgemäßen Kombination tritt bei der photometrischen Bestimmung des Farbstoffs keine Störung durch Ausfällungen oder Trübungen auf.

Für die Durchführung des erfindungsgemäßen Verfahrens eignet sich ein Reagenz zur Bestimmung von Eisen in Körperflüssigkeiten, insbesondere im Serum, enthaltend ein Reduktionsmittel, ein für den Nachweis von Eisen geeignetes Farbstoffsystem, sowie ein oder mehrere Tenside, das dadurch gekennzeichnet ist, daß es einen Fettsäurepolyethylenglykolester, einen Alkanolpolyglykolether und Guanidinhydrochlorid enthält.

Dieses Reagenz eignet sich insbesondere zur Bestimmung von Eisen in lipämischen, hämolytischen und/oder Immunglobulin-reichen Körperflüssigkeiten , insbesondere in Seren.

Die Erfindung wird noch durch folgende Beispiele und Zeichnungen erläutert:

Die Zeichnungen zeigen:

Fig. 1: Zeitliche Änderungen des Meßsignals bei einer Eisenbestimmung mit einem erfindungsgemäßen Reagens

P: Probe

PLW: Probenleerwert

Fig. 2: Aufklarung von lipämischem Serum

A: erfindungsgemäßes Reagens

B: nur Guanidinhydrochlorid

Fig. 3: Einfluß von hämolytischen Seren auf die Wiederfindung

1: erfindungsgemäßes Reagens

2: Reagens gemäß EP 130537

**Beispiel 1**

In einer Humanserumprobe wurde Eisen bestimmt. Dazu wurden die folgenden Reagenzien verwendet:

|  | Probenreagenz | Leerwertreagenz |
|---|---|---|
| Guanidinhydrochlorid | 4,5 mol/l | 4,5 mol/l |
| Acetatpuffer, pH 5,0 | 0,15 mol/l | 0,15 mol/l |
| Iso-Decanol-Polyglykolether | 2,0 % (v/v) | 2,0 % (v/v) |
| Polyethylenglykol-400-monolaurat | 10,0 % (v/v) | 10,0 % (v/v) |
| Ascorbinsäure | 0,023 mol/l | 0,023 mol/l |
| Ferrozin® * | 1,6 mmol/l | -- |

* 3-(2-pyridyl)-5,6-bis(4-phenylsulfonsäure)-1,2,4-triazin-dinatriumsalz

Die Eisenbestimmung wurde in Küvetten mit einer Schichtdicke von 1 cm durchgeführt.

Die Reagenzien wurden in zwei Küvetten pipettiert, wobei in einer Küvette die Probe und in der zweiten Küvette der Probenleerwert gemessen wurde.

|  | Probenleerwert | Probe |
|---|---|---|
| Leerwertreagenz | 1,0 ml | - |
| Probenreagenz | - | 1,0 ml |
| Probe | 0,2 ml | 0,2 ml |

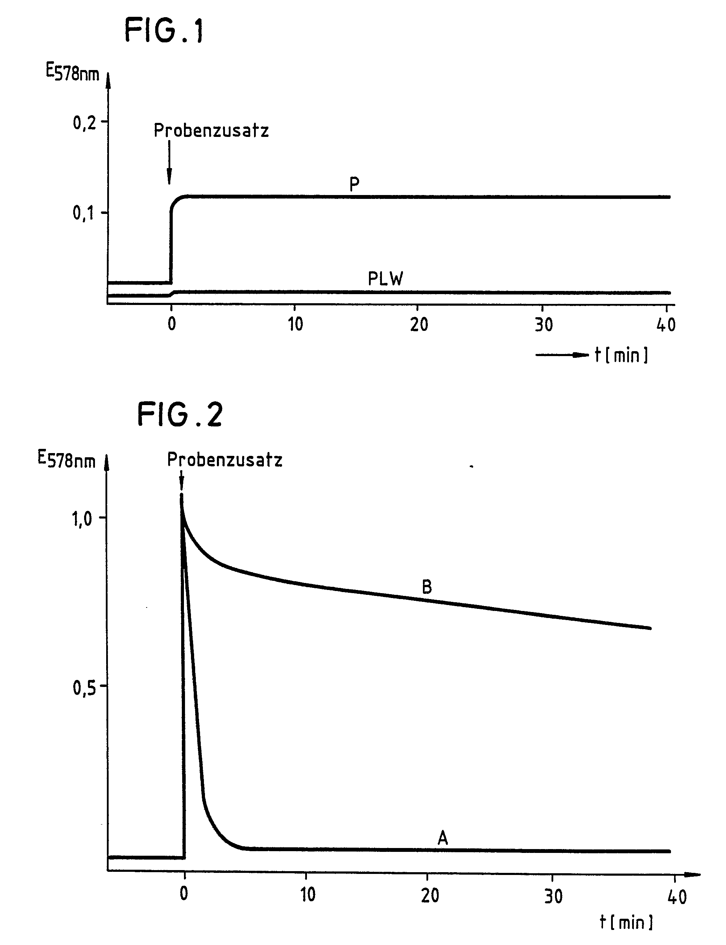

Die Lösungen wurden jeweils in der Küvette gemischt und 10 Minuten bei 25°C inkubiert. Innerhalb von weiteren 30 Minuten wurde bei einer Wellenlänge von 562 nm bzw. Hg 578 nm die Extinktion des Probenleerwerts gegen eine Mischung aus 1 ml Leerwertreagenz, 0,2 ml destilliertes Wasser ($E_{PL}$) sowie die der Probe gegen eine Mischung aus 1 ml Probenreagenz und 0,2 ml destilliertes Wasser ($E_P$) gemessen. Der Wert für die Extinktiondifferenz ergibt sich dann als $E = E_P - E_{PL}$. Der zeitliche Verlauf des Proben- bzw. Probenleerwertsignals ist der Fig. 1 zu entnehmen.

Der Eisengehalt in der Probe berechnet sich nach den folgenden Gleichungen:

Fe ($\mu$g/dl) = E x 1330 für die Messung bei 578 nm und

Fe ($\mu$g/dl) = E x 1220 für die Messung bei 562 nm.

## Beispiel 2

Es wurde ein stark lipämisches Serum verwendet, das 1900 mg/dl Triglyceride enthielt. Dazu wurden 200 $\mu$l des Serums mit 1 ml Leerwertreagenz gemäß Beispiel 1 vermischt und der Extinktionsverlauf bei 25°C und 578 nm gemessen. Das Ergebnis ist der Kurve A in Fig. 2 zu entnehmen. Weiterhin wurden zum Vergleich 200 $\mu$l des Serums mit einem Reagenz, das aus 4,5 M Guanidinhydrochlorid, 0,15 M Acetatpuffer, pH 5,0 und 0,023 M Ascorbinsäure bestand, vermischt. Auch hier wurde der Extinktionsverlauf bei 25°C und 578 nm gemessen. Das Ergebnis ist der Kurve B in Fig. 2 zu entnehmen. Wie ein Vergleich der beiden Kurven A und B zeigt, wird mit einem Reagenz, das nur Acetatpuffer und Guanidiniumhydrochlorid enthält, eine sehr begrenzte und darüberhinaus zeitlich instabile Aufklarung erreicht, während mit dem erfindungsgemäßen Reagenz das lipämische Serum innerhalb von ca. 7 Minuten vollständig aufgeklart werden kann, wobei die Aufklarung über einen Zeitraum von mehr als 30 Minuten bestehen bleibt.

## Beispiel 3

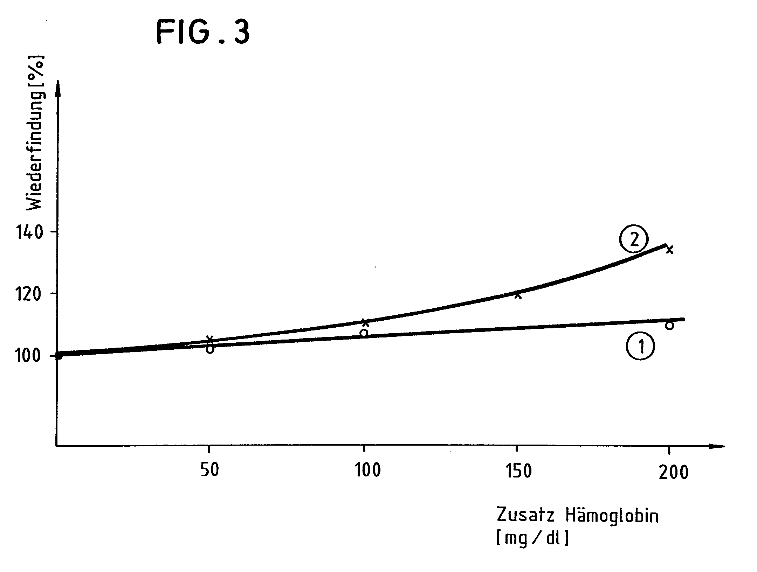

Ein nichthämolytisches Serum wurde mit Hämoglobin zu Konzentrationen bis 200 mg/dl stufenweise aufgestockt und einmal mit dem erfindungsgemäßen Reagenz und zum anderen mit einem Reagenz, wie es in EP 130 537 beschrieben ist, der Eisengehalt, wie im Beispiel 1 beschrieben, bestimmt. Das Ergebnis ist der Fig. 3 zu entnehmen. Dabei zeigt sich, daß mit dem erfindungsgemäßen Reagenz der Eisengehalt selbst bei sehr hohen Hämoglobinwerten nur unwesentlich zu hoch wiedergefunden wird, während bei dem bekannten Reagenz schon leicht erhöhte Hämoglobinwerte stören.

**Beispiel 4**

Es wurde der Eisengehalt in 3 verschiedenen Seren bestimmt, die von Gammopathie-Patienten stammen. Bei der Bestimmung mit dem erfindungsgemäßen Reagenz wird ein Meßsignal erreicht, das sich 60 Minuten lang nicht mehr ändert, was bedeutet, daß während dieses Zeitraums keine Eintrübung auftritt. Demgegenüber zeigt sich bei Verwendung des bekannten Reagenzes nach Beginn der Farbbildungsreaktion eine schnelle Eintrübung, die zu einer Störung bei der photometrischen Messung führt.

**Beispiel 5**

Es wurde die Löslichkeit der eingesetzten Reagenzien, die Ablösung des Eisens von seinem Trägerprotein, die Aufhellung lipämischer Seren, die Eintrübung durch Gammopathieseren und die Störung durch hämolytische Seren überprüft. Die Ergebnisse sind der Tabelle I zu entnehmen.

| Reagenszusätze | Löslichkeit des Reagens | Eisen-Ablösung | Aufhellung lipämischer Seren | Eintrübung Gammopathie-Seren | Störung durch hämolytische Seren |
|---|---|---|---|---|---|
| 1. Anionisches Detergens (sek. Alkansulfonat, Hostapur) | + | + | +/- | - | - |
| 2. Nichtionisches Detergens (Alkanolpolyglycolether, Oxatal ID104) | - | o | + | o | o |
| 3. Nichtionisches Detergens (Fettsäure-polyethylenglycol-ester, PEG-400-monolaurat) | + | - | - | o | o |
| 4. Guanidinhydrochlorid | + | + | - | + | + |
| 5. Kombination 1 + 2 | + | + | + | - | - |
| 6. Kombination 2 + 3 | + | - | + | o | o |
| 7. Kombination 1 + 4 | - | o | o | o | o |
| 8. Kombination 2 + 4 | - | + | o | o | o |
| 9. Kombination 3 + 4 | + | + | - | o | o |
| 10. Kombination 1 + 2 + 4 | - | o | o | o | o |
| 11. Kombination 1 + 3 + 4 | - | o | o | o | o |
| 12. Kombination 2 + 3 + 4 | + | + | + | + | + |

+ bedeutet, daß das Kriterium erfüllt ist (z.B. Reagenz ist löslich, keine Eintrübung durch Gammopathie-Seren)

- bedeutet, daß das Kriterium nicht erfüllt ist (z.B. unbefriedigende Eisenablösung, nicht befriedigende Wiederfindung bei hämolytischen Proben)

o bedeutet, nicht gemessen, da Reagenz bereits durch Nichterfüllung anderer Kriterien ungeeignet ist.

EP 0 306 859 A1

Die Löslichkeit des Reagenses wurde visuell beurteilt. In den mit - gekennzeichneten Zeilen traten entweder starke Trübungen oder Ausfällungen in einem Konzentrationsbereich von 1 bis 15 Gew.-% auf.

Die Beurteilung der Eisenablösung erfolgte durch Eisenbestimmung, wie in Beispiel 1 beschrieben. Sie galt dann als gegeben, wenn bei Einsatz eines Kontrollserums als Probe der Eisengehalt zu mehr als 95 % wiedergefunden wird.

Eine ausreichende Aufhellung lipämischer Seren ist gewährleistet, wenn bei Durchführung eines Versuchs gemäß Beispiel 2 die Extinktion $E_{578}$ kleiner als 0,05 nach spätestens 7 Minuten bei 25° C erreicht wird und über weitere 30 Minuten konstant bleibt.

Die Eintrübung durch Gammopathieseren wurde analog Beispiel 4, die Wiederfindung bei hämolytischen Seren analog Beispiel 3 beurteilt.

**Ansprüche**

1. Verfahren zur Bestimmung von Eisen in Körperflüssigkeiten durch Freisetzung des gebundenen Eisens, Reduktion zu $Fe^{2+}$, Zugabe eines für den Nachweis von Eisen geeigneten Farbsystems und photometrische Messung in einer tensidhaltigen Probelösung, **dadurch gekennzeichnet,** daß man der Probelösung Fettsäure-Polyethylenglykol-Ester, Alkanol-Polyglykol-Ether und mindestens 1 Mol/l Guanidinhydrochlorid zusetzt.

2. Verfahren nach Anspruch 1, **dadurch gekennzeichnet,** daß man in der Probelösung durch Zusatz eines Puffers einen pH-Wert von 5 bis 6 einstellt.

3. Verfahren nach Anspruch 2, **dadurch gekennzeichnet,** daß man als Puffer Acetatpuffer in einer Konzentration von 20 bis 500 mmol/l verwendet.

4. Verfahren nach einem der vorhergehenden Ansprüche, **dadurch gekennzeichnet,** daß man 3 bis 6 Mol/l Guanidinhydrochlorid verwendet.

5. Verfahren nach einem der vorhergehenden Ansprüche, **dadurch gekennzeichnet,** daß man als Fettsäure-Polyethylenglykolester Ester, insbesondere Monoester, von linearen Fettsäuren mit 10 bis 14 Kohlenstoffatomen und einem Polyethylenglykol mit einem durchschnittlichen Molekulargewicht von 200 bis 600 verwendet.

6. Verfahren nach Anspruch 5, **dadurch gekennzeichnet,** daß man als Fettsäure Polyethylenglykolester Polyethylenglykolmonolaurat einsetzt.

7. Verfahren nach einem der vorhergehenden Ansprüche, **dadurch gekennzeichnet,** daß man den Fettsäurepolyethylenglykolester in einer Konzentration von 2,5 bis 15 % einsetzt.

8. Verfahren nach Anspruch 7, **dadurch gekennzeichnet,** daß man den Fettsäurepolyethylenglykolester in einer Konzentration von 5 bis 10 % einsetzt.

9. Verfahren nach einem der vorhergehenden Ansprüche, **dadurch gekennzeichnet,** daß man als Alkanol-Polyglykolether den Ether eines linearen oder verzweigtkettigen Alkylalkohols mit 8 bis 12 C-Atomen und eines Polyglykols mit durchschnittlich 3 bis 6 Glykoleinheiten verwendet.

10. Verfahren nach Anspruch 9, **dadurch gekennzeichnet,** daß man als Alkanol-Polyglykolether einen Ether eines Isodecanols mit durchschnittlich 4 Glykoleinheiten pro Molekül verwendet.

11. Verfahren nach einem der vorhergehenden Ansprüche, **dadurch gekennzeichnet,** daß man den Alkanol-Polyglykolether in einer Konzentration von 0,5 bis 5, insbesondere 1 bis 3 % verwendet.

12. Reagenz zur Bestimmung von Eisen in Serum, enthaltend ein Reduktionsmittel, ein für den Nachweis von Eisen geeignetes Farbstoffsystem, ein oder mehrere Tenside, **dadurch gekennzeichnet,** daß es einen Fettsäure-Polyethylenglykolester, einen Alkanol-Polyglykolether und Guanidinhydrochlorid enthält.

## FIG.1

## FIG.2

FIG.3

EP 0 306 859 A1

| | **EINSCHLÄGIGE DOKUMENTE** | | EP 88114386.1 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| D,Y | CLINICAL CHEMISTRY, Vol. 23, Nr. 2, 1977, Washington D.C. H. WILLIAMS et al. "Simultaneous spectrophotometry of $Fe^{2+}$ and $Cu^{2+}$ in serum denaturated with guaniding hydrochloride" Seiten 237-240 <br><br> * Seiten 237-238, linke Spalte, dritt-letzter Absatz * <br><br> -- | 1,4 | G 01 N 33/84 <br> G 01 N 31/22 |
| D,Y | EP - A2 - 0 130 537 (BOEHRINGER MANNHEIM GMBH) <br><br> * Beispiele; Ansprüche * <br><br> -- | 1,5 | |
| Y | US - A - 4 407 962 (TABACCO et al.) <br><br> * Ansprüche; Spalte 3, Zeilen 30-35 * <br><br> ---- | 1,2 | **RECHERCHIERTE SACHGEBIETE (Int. Cl.4)** <br><br> G 01 N |

Der vorliegende Recherchenbericht wurde für alle Patentansprüche erstellt.

| Recherchenort | Abschlußdatum der Recherche | Prüfer |
|---|---|---|
| WIEN | 12-12-1988 | TENGLER |